(19) Europäisches Patentamt / European Patent Office / Office européen des brevets

(11) **EP 2 995 333 A1**

(12) **EUROPEAN PATENT APPLICATION**
published in accordance with Art. 153(4) EPC

(43) Date of publication:
**16.03.2016 Bulletin 2016/11**

(21) Application number: **13883960.0**

(22) Date of filing: **10.05.2013**

(51) Int Cl.:
**A61M 5/28** (2006.01)

(86) International application number:
**PCT/JP2013/063210**

(87) International publication number:
**WO 2014/181474 (13.11.2014 Gazette 2014/46)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Terumo Kabushiki Kaisha**
**Tokyo 151-0072 (JP)**

(72) Inventors:
• **OKIHARA, Hitoshi**
  **Fujinomiya-shi**
  **Shizuoka 418-0004 (JP)**
• **YAMASHITA, Arisa**
  **Kanagawa 259-0151 (JP)**

(74) Representative: **Casalonga**
**Casalonga & Partners**
**Bayerstraße 71/73**
**80335 München (DE)**

(54) **PRODUCTION METHOD FOR PRE-FILLED SYRINGE, AND PRE-FILLED SYRINGE PRODUCTION DEVICE**

(57)    According to this production method for a pre-filled syringe (100) and pre-filled syringe production device (10), a syringe assembly (107) having an outer barrel (106) filled with a drug solution (110) is held oriented with the barrel tip (102) at the bottom, the interior of the outer barrel (106) of the syringe assembly (107) is depressurized to a set pressure, and a basal end opening (104) of the outer barrel (106) is sealed with a gasket (112), whereby the gasket (112) is arranged at the sealing location, and the gasket (112) is pushed in towards the barrel tip (102) from the sealing location to an insertion location, at a speed faster than the speed in the gasket (112) would be moved utilizing atmospheric pressure.

FIG. 4A

FIG. 4B

FIG. 4C

FIG. 4D

## Description

Technical Field

The present invention relates to a production method for a pre-filled syringe and a pre-filled syringe production device.

Background Art

During a process of producing a pre-filled syringe, a method for inserting a gasket into an outer barrel has been proposed in which a proximal end opening of an outer barrel is sealed with the gasket with a pressure in the outer barrel being reduced, and the gasket is inserted to a predetermined position in the outer barrel due to a difference between a pressure inside the outer barrel and a pressure outside the outer barrel (atmosphere) (e.g., refer to JP 4-28386 B).

Summary of Invention

Technical Problem

In the conventional technique described above, when sliding resistance of the gasket to the outer barrel is high, much time is taken (e.g., a few minutes to about several tens of minutes) to move the gasket to a predetermined position due to a difference between a pressure inside the outer barrel and a pressure outside the outer barrel. When much time is taken for the insertion of the gasket, the pressure-reduced state in a space between the gasket and a drug solution is continued for a long time. Therefore, air bubbles might be generated from gas dissolved in the drug solution, and the generated air bubbles might be adhered onto the inner surface of the outer barrel. This might cause a problem in a following foreign matter inspection. When much time is taken for an insertion of the gasket in a process before a process of inspecting an insertion position of the gasket, a time lag is generated until the start of the inspection, resulting in deteriorated production efficiency.

In view of the above problems, the present invention is to provide a production method for a pre-filled syringe, and a pre-filled syringe production device that can quickly insert a gasket to a predetermined position upon inserting the gasket into an outer barrel.

Solution to Problem

In order to attain the foregoing object, the invention of a method according to the subject application is a production method for a pre-filled syringe that includes an outer barrel having a barrel tip and a proximal end opening, a cap sealing the barrel tip, a drug solution filled in the outer barrel, and a gasket that is slidable in the outer barrel, the gasket being in direct contact with an inner peripheral surface of the outer barrel, and sliding resistance of the gasket to the outer barrel being higher than sliding resistance of the gasket to the outer barrel if a liquid lubricant is present between the inner peripheral surface of the outer barrel and an outer peripheral surface of the gasket, the production method comprising: (a) holding a syringe assembly, which includes the barrel tip sealed with the cap and the outer barrel filled with a predetermined amount of the drug solution, with the barrel tip being oriented at a bottom; (b) reducing a pressure in the outer barrel of the syringe assembly held by the (a) to a set pressure; (c) sealing the proximal end opening of the outer barrel with the gasket while the pressure in the outer barrel is at the set pressure, thereby disposing the gasket to a sealing position; and (d) after the (c), pushing the gasket toward the barrel tip with a liquid-tight sliding motion in the outer barrel with a speed higher than a speed upon moving the gasket by utilizing the atmospheric pressure, in at least a part of a process of moving the gasket from the sealing position to an insertion position, the insertion position being closer to the barrel tip than the sealing position, and the insertion position being a position at which a distal end surface of the gasket is apart from a liquid level of the drug solution with a predetermined distance, wherein, at the insertion position, a pressure in a space between the liquid level of the drug solution and the distal end surface of the gasket is approximately equal to the atmospheric pressure.

According to the above production method for the pre-filled syringe of the present invention, the gasket is forcibly pushed into the outer barrel after being disposed at the sealing position relative to the outer barrel, when the sliding resistance of the gasket to the outer barrel is high. Therefore, the gasket can more quickly be inserted to a predetermined position (insertion position) in the outer barrel than the conventional method in which the gasket is moved only by using a differential pressure between the pressure in the outer barrel and the atmospheric pressure. Accordingly, the present invention can prevent the generation of air bubbles of the drug solution, whereby no problem is caused in a foreign matter inspection in a subsequent process. Even when a process of inspecting the insertion position of the gasket is carried out, a time lag until the start of the inspection is short, whereby production efficiency can be enhanced.

In the above production method for a pre-filled syringe, the maximum sliding resistance when the gasket slides from the sealing position to the insertion position in the outer barrel with a speed of 200 mm/min with the cap not attached to the barrel tip and with the drug solution not filled in the outer barrel is 3 N or higher, and in the (d), the gasket is pushed into the outer barrel with a speed equal to or higher than 50 mm/min. The gasket can quickly be inserted into the outer barrel by pushing the gasket with the speed described above.

In the above production method for a pre-filled syringe, in the (d), the gasket is pushed to the insertion position from the sealing position by controlling a position of a pushing member that is insertable into the outer barrel. With this pushing process of pushing the gasket by the pushing member, the gasket can quickly and surely be inserted to the insertion position with a desired speed.

In the above production method for a pre-filled syringe, in the (d), the gasket is pushed to the insertion position from the sealingpositionby controlling a position of a pushingmember that is insertable into the outer barrel. With this, the gasket can surely be stopped at the insertion position.

The above production method for a pre-filled syringe may further include: (e) holding the gasket at an opposite position at which the distal end surface of the gasket opposes the proximal end opening of the outer barrel in the syringe assembly that is oriented with the barrel tip at the bottom, wherein, in the (d), the gasket located at the opposite position is pushed to the insertion position beyond the sealing position in the outer barrel by a continuous motion of the pushing member that is insertable into the outer barrel. Accordingly, the control for the motion of the pushing member becomes simple, whereby production efficiency can be enhanced.

In the above production method for a pre-filled syringe, in the (d), only the gasket may be in contact with a portion of the inner peripheral surface of the outer barrel at the side closer to the proximal end opening than to the drug solution. This can prevent damage on the inner peripheral surface of the outer barrel.

In the above production method for a pre-filled syringe, the set pressure is a pressure calculated based on a distance from a point where the gasket inserted from the proximal end opening first forms a liquid-tight seal with the inner peripheral surface of the outer barrel or from a proximal end surface of the outer barrel to a liquid level of the drug solution filled in the outer barrel that is oriented with the barrel tip at the bottom, and a distance from a most distal point where a liquid-tight seal is formed between the gasket at the insertion position and the inner peripheral surface of the outer barrel to the liquid level of the drug solution filled in the outer barrel that is oriented with the barrel tip at the bottom. This can surely allow the space between the drug solution and the gasket to have a pressure almost equal to the atmospheric pressure, when the gasket is inserted to the insertion position.

The invention of a device according to the subject application is a pre-filled syringe production device for producing a pre-filled syringe that includes an outer barrel having a barrel tip and a proximal end opening, a cap sealing the barrel tip, a drug solution filled in the outer barrel, and a gasket that is slidable in the outer barrel, the gasket being in direct contact with an inner peripheral surface of the outer barrel, and sliding resistance of the gasket to the outer barrel being higher than sliding resistance of the gasket to the outer barrel if a liquid lubricant is present between the inner peripheral surface of the outer barrel and an outer peripheral surface of the gasket, the pre-filled syringe production device comprising: a syringe holdingmember configured to hold a syringe assembly, which includes the barrel tip sealed with the cap and the outer barrel filled with a predetermined amount of the drug solution, with the barrel tip oriented at the bottom; a pressure control mechanism configured to reduce a pressure in the outer barrel of the syringe assembly to a set pressure; a gasket arranging mechanism configured to dispose the gasket at a sealing position by sealing the proximal end opening of the outer barrel with the gasket; and a pushing mechanism configured to push the gasket toward the barrel tip in the outer barrel, wherein the pushing mechanism is configured to push the gasket toward the barrel tip with a liquid-tight sliding motion in the outer barrel with a speed higher than a speed upon moving the gasket by utilizing the atmospheric pressure, in at least a part of a process of moving the gasket from the sealing position to an insertion position that is closer to the barrel tip than the sealing position and where a distal end surface of the gasket is apart from a liquid level of the drug solution with a predetermined distance, and wherein, at the insertion position, a pressure in a space between the liquid level of the drug solution and the distal end surface of the gasket is approximately equal to the atmospheric pressure.

According to the above pre-filled syringe production device of the present invention, the gasket can more quickly be inserted to a predetermined position (insertion position) in the outer barrel than the conventional method in which the gasket is moved only by using a differential pressure between the pressure in the outer barrel and the atmospheric pressure. Accordingly, no problem is caused in a foreign matter inspection in a subsequent process. Even when a process of inspecting the insertion position of the gasket is carried out, a time lag until the start of the inspection is short, whereby production efficiency can be enhanced.

In the above pre-filled syringe production device, the maximum sliding resistance when the gasket slides from the sealing position to the insertion position in the outer barrel with a speed of 200 mm/min with the cap not attached to the barrel tip and with the drug solution not filled in the outer barrel may be 3 N or higher, and wherein the pushing mechanism is configured to push the gasket into the outer barrel with a speed equal to or higher than 50 mm/min. The gasket can quickly be inserted into the outer barrel by pushing the gasket with the speed described above.

In the above pre-filled syringe production device, the pushing mechanismmay include a pushing member that is insertable into the outer barrel and is configured to push the gasket to the insertion position from the sealing position by controlling the position of a pushing member. With this pushing process of pushing the gasket by the pushingmember, the gasket can quickly and surely be inserted to the insertion position with a desired speed.

In the above pre-filled syringe production device, the pushing mechanism is configured to push the gasket to the insertion position from the sealing position by controlling a position of a pushing member that is insertable into the outer barrel. With this, the gasket can surely be stopped at the insertion position.

The above pre-filled syringe production device may further include a gasket holdingmember that is configured hold the gasket at an opposite position at which the distal end surface of the gasket opposes the proximal end opening of the outer barrel in the syringe assembly that is oriented with the barrel tip at the bottom, wherein a pushing member that is insertable into the outer barrel and that serves as both the gasket arranging mechanism and the pushing mechanism, the pushing member configured to push the gasket located at the opposite position to the insertion position beyond the sealing position in the outer barrel in a continuous motion of the pushing member. With this, the gasket is pushed into the outer barrel from the opposite position to the insertion position with one pushing motion. Accordingly, the control for the motion of the pushing member becomes simple, whereby production efficiency can be enhanced.

In the above pre-filled syringe production device, only the gasket may be in contact with a portion of the inner peripheral surface of the outer barrel at the side closer to the proximal end opening than to the drug solution when the gasket is moved to the insertion position from the sealing position. This can prevent damage on the inner peripheral surface of the outer barrel.

In the above pre-filled syringe production device, the set pressure is a pressure calculated based on a distance from a point where the gasket inserted from the proximal end opening first forms a liquid-tight seal with the inner peripheral surface of the outer barrel or from a proximal end surface of the outer barrel to a liquid level of the drug solution filled in the outer barrel that is oriented with the barrel tip at the bottom, and a distance from a most distal position where a liquid-tight seal is formed between the gasket at the insertion position and the inner peripheral surface of the outer barrel to the liquid level of the drug solution filled in the outer barrel that is oriented with the barrel tip at the bottom. This can surely allow the space between the drug solution and the gasket to have a pressure almost equal to the atmospheric pressure, when the gasket is inserted to the insertion position.

Advantageous Effects of Invention

According to the production method for a pre-filled syringe and the pre-filled syringe production device of the present invention, a gasket can quickly be inserted to a predetermined position upon inserting the gasket into an outer barrel.

Brief Description of Drawings

Fig. 1 is a longitudinal sectional view illustrating an example of a configuration of a pre-filled syringe.
Fig. 2 is a view illustrating a schematic configuration of a pre-filled syringe production device according to a first embodiment of the present invention.
Fig. 3 is a longitudinal sectional view illustrating a gasket holding member holding a gasket.
Fig. 4A is a first view for describing an operation of the pre-filled syringe production device illustrated in Fig. 2, Fig. 4B is a second view for describing the operation of the pre-filled syringe production device illustrated in Fig. 2, Fig. 4C is a third view for describing the operation of the pre-filled syringe production device illustrated in Fig. 2, and Fig. 4D is a fourth view for describing the operation of the pre-filled syringe production device illustrated in Fig. 2.
Fig. 5 is a view illustrating a schematic configuration of a pre-filled syringe production device according to a second embodiment of the present invention.
Fig. 6A is a first view for describing an operation of the pre-filled syringe production device illustrated in Fig. 5, Fig. 6B is a second view for describing the operation of the pre-filled syringe production device illustrated in Fig. 5, and Fig. 6C is a third view for describing the operation of the pre-filled syringe production device illustrated in Fig. 5.
Fig. 7A is a fourth view for describing the operation of the pre-filled syringe production device illustrated in Fig. 5, Fig. 7B is a fifth view for describing the operation of the pre-filled syringe production device illustrated in Fig. 5, and Fig. 7C is a sixth view for describing the operation of the pre-filled syringe production device illustrated in Fig. 5.

Description of Embodiments

Preferable embodiments of a production method for a pre-filled syringe, and a pre-filled syringe production device according to the present invention will be described below with reference to the accompanying drawings.

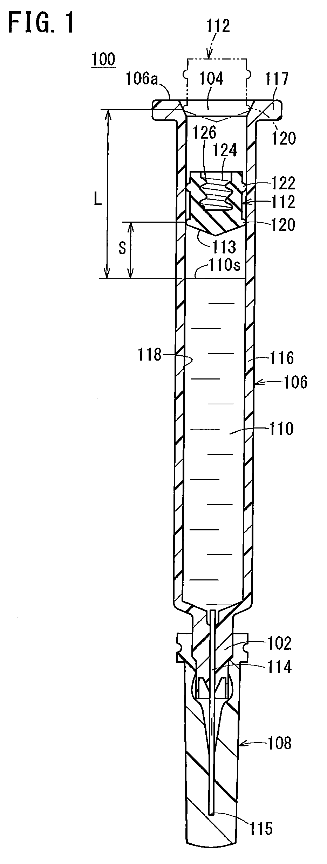

Fig. 1 is a longitudinal sectional view illustrating an example of a configuration of a pre-filled syringe 100. This pre-filled syringe 100 includes a cylindrical outer barrel 106 having a barrel tip 102 and a proximal end opening 104, a cap 108 sealing the barrel tip 102, a drug solution 110 filled in the outer barrel 106, and a gasket 112 that can slide in the outer barrel 106. This pre-filled syringe 100 is configured as a syringe with a needle having a needle 114 fixed on the barrel tip 102. The pre-filled syringe 100 may not be provided with the needle 114.

An internal cavity 118 is formed in an outer barrel body 116 forming a body of the outer barrel 106. The drug solution 110 is filled in this internal cavity 118. The barrel tip 102 projects from the distal end of the outer barrel body 116 toward the distal end with its diameter being narrower than the outer barrel body 116. A proximal end of the hollow needle 114 having a sharp needle tip 115 at its distal end is fixed to the barrel tip 102. A flange 117 projecting outward is provided at the proximal end of the outer barrel body 116. The proximal end opening 104 is formed inside the flange 117.

The cap 108 is formed to be a bottomed cylinder with its proximal end open, and is made of an elastic material. The needle tip 115 of the needle 114 is inserted into the distal end of the cap 108 with the cap 108 being attached to the outer barrel 106. Thus, the opening at the distal end of the needle 114 is sealed, whereby the drug solution 110 is not leaked from the opening at the distal end in an unused state in which the cap 108 is attached to the barrel tip 102.

Examples of the material forming the cap 108 include various rubber materials such as a natural rubber, a butyl rubber, an isoprene rubber, a butadiene rubber, a styrene-butadiene rubber, and a silicon rubber; various thermoplastic elastomers such as a polyurethane elastomer, a polyester elastomer, a polyamide elastomer, an olefin elastomer, and a styrene elastomer; or their mixtures.

Examples of the drug solution 110 include injection for an electrolyte imbalance correction such as sodium chloride or potassiumlactate; various drug solutions and various diagnostic agents such as a vitamin, a vaccine, an intravenous antibiotic, a steroid, an insulin, an antibody drug, a protease inhibitor, a fat emulsion, various protein preparations, an anticancer, an anesthetic, a stimulant, and a narcotic.

The gasket 112 is inserted into the outer barrel 106. A distal end surface 113 of the gasket 112 has a taper shape that becomes narrower toward the distal end. The distal end surface 113 of the gasket 112 is apart from a liquid level 110s of the drug solution 110, when the barrel tip 102 is oriented at the bottom. In other words, a space with a predetermined volume is formed between the liquid level 110s of the drug solution 110 and the distal end surface 113 of the gasket 112, when the barrel tip 102 is oriented at the bottom. A pressure of this space is almost equal to the atmospheric pressure.

Two annular seal projections 120 and 122 are formed on an outer periphery of the gasket 112 with a space in the axial direction. In the state in which the gasket 112 is inserted into the outer barrel 106, the two seal projections 120 and 122 are in close contact with the inner peripheral surface of the outer barrel 106. With this, the gasket 112 can slide in the outer barrel 106 in a liquid-tight manner. The gasket 112 may be configured such that a coating film for reducing sliding resistance is formed on an outer surface of a gasket base material. Examples of the coating film include fluorine resins such as polytetrafluoroetylene (PTFE), tetrafluoroethylene-perfluoroalkylvinylether copolymer (PFA), fluorinated ethylene-propylene copolymer (FEP), polychlorotrifluoroethylene (PCTFE), and poly(vinylidene fluoride)(PVDF); silicon resins made of liquid coating containing reactive silicon oil as a main component, such as silicon polymer coating film; poly-para-xylylene; and diamond-like carbon.

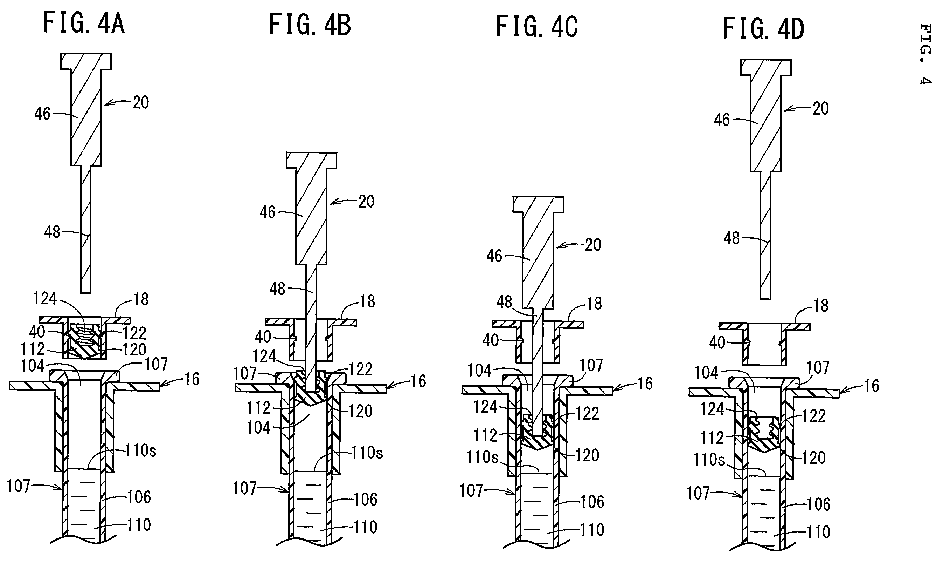

A liquid lubricant (e.g., silicon oil) is not applied on the inner peripheral surface of the outer barrel 106 and the outer peripheral surface of the gasket 112, and the outer periphery (the seal projections 120 and 122) of the gasket 112 is in direct contact with the inner periphery of the outer barrel 106. Therefore, the sliding resistance of the gasket 112 to the outer barrel 106 is high. Even if the gasket 112 is configured such that a coating film reducing the sliding resistance is formed on the outer surface of the gasket base material, the coating film forms a part of the gasket 112. Therefore, this configuration means that the outer periphery of the gasket 112 is in direct contact with the inner peripheral surface of the outer barrel 106. The maximum sliding resistance when the gasket 112 slides in the outer barrel 106 with a speed of 200 mm/min from a later-described sealing position (the position of the gasket 112 illustrated in Fig. 4B) to an insertion position (the position of the gasket 112 illustrated in Fig. 1) with the state in which the cap 108 is not attached to the outer barrel 106 and the drug solution 110 is not filled in the outer barrel 106 is 3 N (Newton) or higher, for example.

A fitted concave portion 124 that is open at the proximal end is formed on the gasket 112. A female screw 126 is formed on the inner periphery of the fitted concave portion 124 that can be threaded to a distal end of a pusher not illustrated. The materials described above as the examples of the materials for the cap 108 can be used for the gasket 112.

Next, a device for producing the pre-filled syringe 100 configured as described above, especially a device for performing a process of inserting the gasket 112 into the outer barrel 106 filled with the drug solution 110, will be described according to each embodiment of the present invention.

First Embodiment

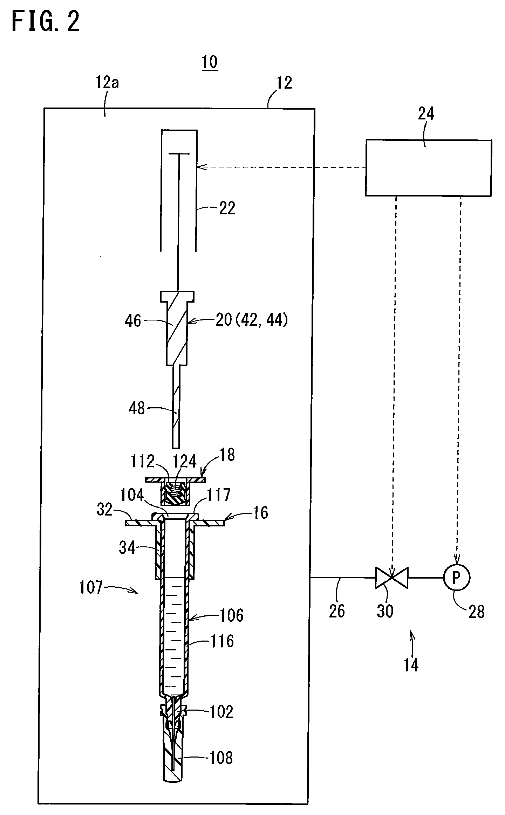

Fig. 2 is a view illustrating a schematic configuration of a pre-filled syringe production device 10 (hereinafter abbreviated to a "production device 10") according to a first embodiment of the present invention. The production device 10 includes a chamber 12, a pressure control mechanism 14, a syringe holding member 16, a gasket holding member 18, a pushing member 20, an actuator 22, and a control unit 24.

The chamber 12 has a storage chamber 12a that can store an assembly having the barrel tip 102 sealed with the cap 108 and having the outer barrel 106 filled with the drug solution 110 with the barrel tip 102 being oriented at the bottom, and the gasket 112 (hereinafter referred to as a "syringe assembly 107"). The pressure in the storage chamber 12a can be reduced and also can be returned to the atmospheric pressure from the reduced state under the action of the pressure control mechanism 14.

Although not illustrated in Fig. 2, the chamber 12 has an opening into which the syringe assembly 107 and the gasket 112 are supplied and from which the pre-filled syringe 100 formed by inserting the gasket 112 into the syringe assembly 107 is carried out and a door for opening and closing the opening. Fig. 2 illustrates a set of the syringe assembly 107 and the gasket 112. However, the chamber 12 may be configured to be capable of storing a plurality of sets of the syringe assembly 107 and the gasket 112.

The pressure control mechanism 14 includes an air line 26 with one end connected to the chamber 12, a vacuum source 28 connected to the other end of the air line 26, and a control valve 30 mounted on the air line 26. The vacuum source 28 is not particularly limited. A vacuum pump or a combination of an ejector and a positive pressure source can be used, for example. A solenoid valve can be used for the control valve 30, for example.

Under the control of the control unit 24, the vacuum source 28 in the pressure control mechanism 14 is operated, and the control valve 30 is opened, whereby air in the chamber 12 (storage chamber 12a) is sucked via the air line 26. Thus, the pressure control mechanism 14 can reduce the pressure in the chamber 12 to a desired negative pressure. The pressure control mechanism 14 can also return the pressure in the chamber 12 to the atmospheric pressure by stopping the operation of the vacuum source 28. An atmosphere open port may be provided on the control valve 30. When the port of the control valve 30 communicating with the vacuum source 28 is shut off, the atmosphere open port may be opened to allow the chamber 12 to be open to the atmosphere to return the pressure in the chamber 12 to the atmospheric pressure. Alternatively, an atmosphere open line and the control valve 30 may separately be provided to the chamber 12.

The syringe holding member 16 is configured to be capable of holding the syringe assembly 107 with the barrel tip 102 being oriented at the bottom. The syringe holding member 16 in this figure has a support plate 32 and a holding cylinder 34 projecting downward from the support plate 32. The inner diameter of the holding cylinder 34 is slightly larger than the outer diameter of the outer barrel body 116 of the outer barrel 106, so that the outer barrel 106 can be inserted into the holding cylinder 34 from the top. When the flange 117 of the outer barrel 106 is in contact with (hooked with) the top surface of the support plate 32, the syringe assembly 107 is held suspended by the syringe holding member 16.

The syringe holding member 16 thus configured may normally be fixed in the chamber 12, or may be detachable from the chamber 12. In the case where the chamber 12 stores a plurality of sets of the syringe assembly 107 and the gasket 112, a number of syringe holding members 16 corresponding to the number of the sets are provided. In this case, a plurality of plural holding cylinders 34 may be provided on one support plate 32.

The gasket holding member 18 holds the gasket 112 at an opposite position (the position of the gasket 112 illustrated in Fig. 2) where the distal end surface 113 of the gasket 112 opposes the proximal end opening 104 of the outer barrel 106 in the syringe assembly 107 with the barrel tip 102 being oriented at the bottom. The gasket holding member 18 is arranged above the syringe holding member 16. The gasket holding member 18 illustrated in this figure is arranged not to be in contact with, i.e., arranged to form a space with, the outer barrel 106 of the syringe assembly 107 held by the syringe holding member 16.

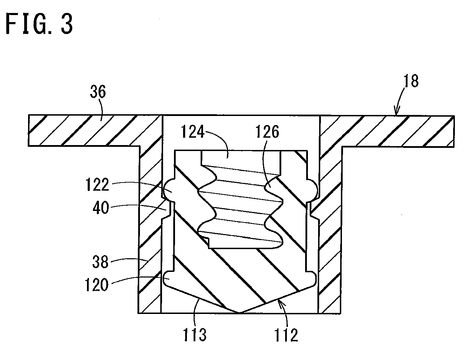

Fig. 3 is a longitudinal sectional view illustrating the gasket holding member 18 holding the gasket 112. The gasket holding member 18 includes a plate portion 36 and a cylindrical holder portion 38 that projects downward from the plate portion 36 and can hold the gasket 112 inside. The holder portion 38 is configured to prevent the gasket 112 from falling due to the force of gravity as well as to allow the gasket 112 to be detached when the gasket 112 is pressed from the proximal end.

The gasket holding member 18 illustrated in this figure is provided with engaging projections 40 projecting inward from the inner peripheral surface of the holder portion 38. In Fig. 3, the engaging projections 40 are formed at the positions for engaging the seal projections 122 at the proximal end of the gasket 112. However, the engaging projections 40 may be formed at the position for engaging the seal projections 120 at the distal end. The engaging projection 40 may be formed to have an annular shape, or a plurality of engaging projections 40 may be formed in the circumference direction with a space. A engaging concave portion that can engage one or both of the seal projections 120 and 122 may be formed on the inner peripheral surface of the holder portion 38, instead of the engaging projection 40.

The gasket holding member 18 thus configured may normally be fixed in the chamber 12, or may be detachable from the chamber 12. In the case where the chamber 12 stores a plurality of sets of the syringe assembly 107 and the gasket 112, a number of gasket holding members 18 corresponding to the number of the sets are provided. In this case, a plurality of the holding cylinders 34 may be provided on one support plate 36.

In the present embodiment, the pushing member 20 has a function of pushing the gasket 112 held by the gasket holding member 18 toward the syringe assembly 107 held by the syringe holding member 16 to arrange the gasket 112 at the proximal end opening 104 of the outer barrel 106, and to further push the gasket 112 in the outer barrel 106 toward the distal end. Therefore, the pushing member 20 has both a function as a gasket arranging mechanism 42 for sealing the proximal end opening 104 of the outer barrel 106 with the gasket 112 and a function as a pushing mechanism 44 for pushing the gasket 112 into the outer barrel 106 toward the barrel tip 102.

The pushing member 20 has a base portion 46 with relatively a large diameter and a pin portion 48 extending downward from the lower end of the base portion 46 and having relatively a small diameter. The pushing member 20 can be inserted into the outer barrel 106 from the proximal end opening 104 of the outer barrel 106 of the syringe assembly 107 held by the syringe holding member 16.

The thickness (outer diameter) of the pin portion 48 of the pushing member 20 is smaller than the inner diameter of the fitted concave portion 124 formed on the gasket 112. Therefore, the distal end of the pin portion 48 can be inserted into the fitted concave portion 124. The thickness (outer diameter) of the base portion 46 of the pushing member 20 is smaller than the inner diameter of the holder portion 38 of the gasket holding member 18. Therefore, the base portion 46 can be inserted into the holder portion 38. The length of the pin portion 48 of the pushing member 20 is preferably larger than the length (depth) of the fitted concave portion 124 of the gasket 112. With this, not the proximal end surface of the gasket 112 but the bottom surface of the fitted concave portion 124 is pushed, whereby the gasket 112 can be inserted without being compressed in the axial direction.

The actuator 22 moves the pushing member 20 in the vertical direction under the control of the control unit 24. The actuator 22 can move the pushing member 20 downward with a speed of 50 to 20000 mm/min. The actuator 22 is not particularly limited. Examples of the actuator include a fluid pressure cylinder device, a linear motor, and a direct operated mechanism using a rack and pinion and a rotation motor.

An actuator that can accurately control the operating positon of an output portion serving as a movable portion under the control of the control unit 24 is preferably used for the actuator 22. In Fig. 2, the lower end of the actuator 22 is connected to the pushing member 20. However, another member or a power transmission mechanism that can transmit a motion of the actuator 22 to the pushing member 20 may be present between the actuator 22 and the pushing member 20.

In Fig. 2, the actuator 22 is arranged in the chamber 12. However, a body (e.g., a cylinder portion of a fluid pressure cylinder device) of the actuator 22 may be arranged outside the chamber 12, and the output portion (e.g., a rod portion of the fluid pressure cylinder) of the actuator 22 may penetrate through a wall of the chamber 12 in an airtight manner.

The production device 10 according to the present embodiment is basically configured as described above, and its operation and effect will next be described. A process of inserting the gasket 112 to a predetermined positon (insertion position) in the outer barrel 106 of the syringe assembly 107 in the production process of the pre-filled syringe 100 will especially be described. The processes below carried out by the production device 10 are performed under the control of the control unit 24. However, the embodiment is not limited thereto, and some processes may manually be performed by an operator.

As illustrated in Fig. 4A, the syringe assembly 107 having the barrel tip 102 sealed with the cap 108 and having the outer barrel 106 filled with a predetermined amount of the drug solution 110 is held in the chamber 12 with the barrel tip 102 being oriented at the bottom, and the gasket 112 is held at the opposite position (just above the proximal end opening 104) opposite to the proximal end opening 104 of the outer barrel 106. Specifically, the syringe assembly 107 is held by the syringe holding member 16, and the gasket 112 is held by the gasket holding member 18. After the syringe assembly 107 and the gasket 112 are held, the door of the chamber 12 is closed, and the inside of the chamber 12 (storage chamber 12a) is formed as an airtight space.

Then, the pressure in the outer barrel 106 of the syringe assembly 107 held by the syringe holding member 16 is reduced to a set pressure. Specifically, the vacuum pump and the control valve 30 are operated under the control of the control unit 24 to evacuate the chamber 12 to the set pressure, whereby the pressure in the outer barrel 106 (the space above the liquid level 110s of the drug solution 110 in the outer barrel 106) is reduced to the set pressure.

The set pressure is a pressure set such that the pressure in the space between the liquid level 110s of the drug solution 110 and the distal end surface 113 of the gasket 112 becomes almost equal to the atmospheric pressure (760 mmHg) when the gasket 112 is inserted to the insertion position in the outer barrel 106.

The set pressure described above can be calculated based on a distance L (see Fig. 1) from the position where the gasket 112 inserted from the proximal end opening 104 of the outer barrel 106 first forms a liquid-tight seal with the inner peripheral surface of the outer barrel 106 to the liquid level 110s of the drug solution 110 filled in the outer barrel 106 that is oriented with the barrel tip 102 at the bottom, and a distance S (see Fig. 1) from the extreme distal end (in the present embodiment, the position of the seal projection 120 at the distal end) where the liquid-tight seal is formed between the gasket 112 at the insertion position and the inner peripheral surface of the outer barrel 106 to the liquid level 110s of the drug solution 110 filled in the outer barrel 106 that is oriented with the barrel tip 102 at the bottom. Specifically, when the set pressure is defined as P, P can be calculated from the equation (1) below.

$$P = (S/L) \times 760 \text{ mmHg} \dots (1)$$

When the position where the gasket 112 inserted from the proximal end opening 104 of the outer barrel 106 first forms a liquid-tight seal with the inner peripheral surface of the outer barrel 106 is almost equal to the proximal end surface 106a of the outer barrel 106, the distance from the proximal end surface 106a of the outer barrel 106 to the liquid level 110s of the drug solution 110 filled in the outer barrel 106 that is oriented with the barrel tip 102 at the bottom may be defined as the distance L.

After the pressure in the outer barrel 106 is reduced to the set pressure, the proximal end opening 104 of the outer barrel 106 is sealed with the gasket 112 with the set pressure being maintained. Specifically in the present embodiment, the gasket 112 held by the gasket holding member 18 is pushed downward by the pushing member 20, while controlling the position of the pushing member 20 by driving the actuator 22 to move the pushing member 20 downward under the control of the control unit 24.

With this, force received by the gasket 112 from the pushing member 20 is larger than the holding force (engagement force between the seal projections 122 and the engaging projections 40) on the gasket 112 by the gasket holdingmember 18. Therefore, the gasket 112 is detached from the gasket holding member 18. The gasket 112 detached from the gasket holding member 18 is further pushed downward by the pushing member 20 to be inserted into the proximal end opening 104 of the outer barrel 106 as illustrated in Fig. 4B, whereby the gasket 112 is arranged at the sealing position.

Then, the gasket 112 is pushed to the insertion position in the outer barrel 106 with the pressure-reduced state with the set pressure being maintained as illustrated in Fig. 4C. Specifically, the gasket 112 is pushed downward by the pushing member 20, while controlling the position of the pushing member 20 by driving the actuator 22 to move the pushing member 20 downward under the control of the control unit 24. In this case, the gasket 112 is pushed toward the barrel tip 102 with a liquid-tight sliding motion in the outer barrel 106 with a speed higher than a speed of moving the gasket 112 by utilizing the atmospheric pressure. For example, the gasket 112 is pushed into the outer barrel 106 with a speed of 50 to 20000 mm/min.

In this case, when the gasket 112 is moved to the insertion position (the position illustrated in Fig. 4C) in the outer barrel 106 from the position (the opposite position illustrated in Fig. 4A) where the gasket 112 is held by the gasket holding member 18, the gasket 112 may be pushed into the outer barrel 106 to the insertion position beyond the sealing position with the continuous motion of the pushing member 20 without being stopped at the sealing position (the position illustrated in Fig. 4B).

The maximum speed of pushing the gasket 112 into the outer barrel 106 may be set to an upper limit of an operating speed of the actuator 22. The gasket 112 may be pushed with a speed not less than 20000 mm/min depending on the operating capability of the actuator 22. During the period in which the pushing member 20 pushes the gasket 112 to move the gasket 112 to the insertion position from the sealing position, the moving speed of moving the gasket 112, i.e., the moving speed of the pushing member 20, may be constant or may be changed during the movement.

As illustrated in Fig. 4C, after the pushing member 20 pushes the gasket 112 to the insertion position in the outer barrel 106, the motion of the pushing member 20 is stopped. The pressure in the space between the drug solution 110 and the gasket 112 is increased due to the downward movement of the gasket 112 during the period in which the gasket 112 is moved from the sealing position to the insertion position. When the gasket 112 is inserted to the insertion position, the pressure in the space between the drug solution 110 in the outer barrel 106 and the gasket 112 becomes almost equal to the atmospheric pressure.

After the gasket 112 is inserted to the insertion position, the control valve 30 is opened to allow the chamber 12 to be open to the atmosphere in order to return the pressure in the chamber 12 to the atmospheric pressure under the control action of the control unit 24. After the pressure in the chamber 12 is returned to the atmospheric pressure, the pushing member 20 is moved upward to be pulled out of the outer barrel 106 and the gasket holding member 18 as illustrated in Fig. 4D. Thus, the pre-filled syringe 100 illustrated in Fig. 1 is produced.

As described above, according to the production method for the pre-filled syringe 100 and the production device 10 of the present embodiment, the gasket 112 is forcibly pushed into the outer barrel 106 after being arranged at the sealing position relative to the outer barrel 106, when the sliding resistance of the gasket 112 to the outer barrel 106 is high. Therefore, the gasket 112 can more quickly be inserted to a predetermined position (insertion position) in the outer barrel 106 than the conventional method in which the gasket 112 is moved only by using a differential pressure between the pressure in the outer barrel 106 (the space between the drug solution 110 and the gasket 112) and the atmospheric pressure. Accordingly, the present embodiment can prevent the generation of air bubbles of the drug solution 110, whereby no problem is caused in a foreign matter inspection in a subsequent process. Even when a process of inspecting the insertion position of the gasket 112 is carried out, a time lag until the start of the inspection is short, whereby production efficiency can be enhanced.

In the case where the maximum sliding resistance is 3 N or more upon sliding the gasket 112 with a speed of 200 mm/min in the outer barrel 106 from the sealing position to the insertion position in the state in which the cap 108 is not attached to the barrel tip 102 and the drug solution 110 is not filled in the outer barrel 106, the gasket 112 is pushed into the outer barrel 106 with a speed of 50 mm/min or higher upon inserting the gasket 112 in the outer barrel 106 of the syringe assembly 107. With this motion of pushing the gasket with this speed, the gasket 112 can quickly be inserted into the outer barrel 106.

The production device 10 may perform the operation of pushing the gasket 112 with a speed higher than the speed of moving the gasket 112 by using the atmospheric pressure during the whole process of moving the gasket 112 from the sealing position to the insertion position, or during a part of the process of moving the gasket 112 from the sealingposition to the insertion position.

In the present embodiment, the pressure in the space between the liquid level 110s of the drug solution 110 and the distal end surface 113 of the gasket 112 is almost equal to the atmospheric pressure at the insertion position. Therefore, even when the pressure at the proximal end of the gasket 112, which is inserted to the insertion position in the outer barrel 106, is returned to the atmospheric pressure, the gasket 112 does not move by the difference in the pressure between the distal end and the proximal end of the gasket 112, resulting in that the insertion of the gasket 112 can surely and quickly be completed.

In the present embodiment, the gasket 112 is pushed from the sealing position to the insertion position by the pushing member 20 that can be inserted into the outer barrel 106. Since the gasket 112 is pushed by the pushing member 20 as described above, the gasket 112 can quickly and surely be inserted to the insertion position with a desired speed.

In the present embodiment, the gasket 112 is pushed to the insertion position by controlling the position of the pushing member 20, whereby the gasket 112 can surely be stopped at the insertion position. On the other hand, force exerted on the pushing member 20 upon the arrival of the gasket 112 at the insertion position may be sensed by a force sensor to stop the movement of the pushing member 20. In this case, a pressure equal to or higher than the atmospheric pressure might be applied to the drug solution 110 in the outer barrel 106.

In the present embodiment, the gasket 112 is held at the opposite position where the distal end surface 113 of the gasket 112 opposes the proximal end opening 104 of the outer barrel 106 in the syringe assembly 107 that is oriented with the barrel tip 102 at the bottom, as illustrated in Fig. 4A. As illustrated in Figs. 4B and 4C, the gasket 112 located at the opposite position is pushed into the outer barrel 106 to the insertion position beyond the sealing position with the continuous motion of the pushing member 20. With this, the gasket 112 is pushed into the outer barrel 106 from the opposite position to the insertion position with one pushing motion. Accordingly, the control for the motion of the pushing member 20 becomes simple, whereby production efficiency can be enhanced.

In the present embodiment, when the gasket 112 is moved from the sealing position to the insertion position, only the gasket 112 is in contact with the inner peripheral surface of the outer barrel 106 at the side closer to the proximal end opening 104 than to the drug solution 110. Therefore, the inner peripheral surface of the outer barrel 106 is not damaged.

In the present embodiment, the set pressure is a pressure calculated based on a distance L from the position where the gasket 112 inserted from the proximal end opening 104 of the outer barrel 106 first forms a liquid-tight seal with the inner peripheral surface of the outer barrel 106 to the liquid level 110s of the drug solution 110 filled in the outer barrel 106 that is oriented with the barrel tip 102 at the bottom, and a distance S from the extreme distal end where the liquid-tight seal is formed between the gasket 112 at the insertion position and the inner peripheral surface of the outer barrel 106 to the liquid level 110s of the drug solution 110 filled in the outer barrel 106 that is oriented with the barrel tip 102 at the bottom. This can surely allow the space between the drug solution 110 and the gasket 112 to have the pressure almost equal to the atmospheric pressure, when the gasket 112 is inserted to the insertion position.

In the present embodiment, after being disposed at the sealing position by the pushing member 20, the gasket 112 is further pushed to the insertion position by the pushing member 20. However, the gasket 112 may be pushed to the insertion position with the process described below. Specifically, a pressure mechanism for increasing the pressure in the chamber 12 to a desired pressure equal to or higher than the atmospheric pressure is provided to the production device 10. After the gasket 112 is arranged at the sealing position, the pressure in the chamber 12 is increased to a pressure higher than the atmospheric pressure.

With this, the pressure higher than the atmospheric pressure is applied to the proximal end (upper side) of the gasket 112, so that the gasket 112 is pushed downward by the difference between the pressure higher than the atmospheric pressure and the pressure (reduced pressure) in the space between the drug solution 110 and the gasket 112. After the gasket 112 reaches the insertion position, the chamber 12 is open to the atmosphere to return the pressure in the chamber 12 to the atmospheric pressure, and the movement of the gasket 112 is stopped. The gasket 112 can also quickly be inserted to the insertion position by increasing the pressure in the chamber 12 to a pressure higher than the atmospheric pressure as described above.

In the description above, the pressure in the chamber 12 is maintained to a reduced pressure that is the set pressure during the insertion of the gasket 112 from the sealing position to the insertion position. However, instead of this process, the pressure in the chamber 12 may be returned to the atmospheric pressure until the gasket 112 is moved to the insertion position after the gasket 112 is arranged at the sealing position.

Second Embodiment

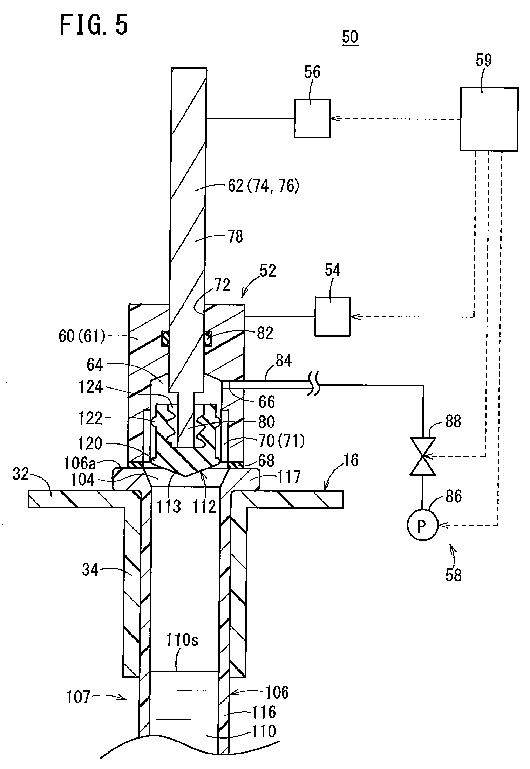

Fig. 5 is a view illustrating a schematic configuration of a pre-filled syringe production device 50 (hereinafter abbreviated to "production device 50") according to a second embodiment of the present invention. This production device 50 includes a syringe holding member 16, a production jig 52, a jig moving mechanism 54, an actuator 56, a pressure control mechanism 58, and a control unit 59.

The syringe holding member 16 in the production device 50 has the same configuration as the syringe holding member 16 in the production device 10 according to the first embodiment. In the production device 50, the syringe holding member 16 is fixed to an appropriate position, or mounted detachably. The production device 50 may be configured to produce a plurality of pre-filled syringes 100 (see Fig. 1) from a plurality of sets of a syringe assembly 107 and a gasket 112. In this case, a number of the syringe holding members 16 corresponding to the number of the sets are provided. In this case, a plurality of the holding cylinders 34 may be provided on one support plate 32.

The production jig 52 includes a jig body 60 that is mounted to cover a proximal end opening 104 of an outer barrel 106, and a pushing member 62 that pushes the gasket 112 downward. The jig body 60 is movable in the vertical direction by an operation of the jig moving mechanism 54. The jig body 60 has a storage space 64 into which the gasket 112 can be stored, and a vent 66 communicating with the storage space 64. The storage space 64 has generally a circular transverse section, and its diameter (inner diameter) is almost equal to or slightly smaller than the diameter (outer diameter) of the gasket 112.

In the present embodiment, the jig body 60 serves as a gasket holding member 61 that holds the gasket 112 at an opposite position where a distal end surface 113 of the gasket 112 opposes the proximal end opening 104 of the outer barrel 106 of the syringe assembly 107 that is oriented with a barrel tip 102 at the bottom.

A first seal member 68 is provided on a lower end of the jig body 60 to enclose a lower end opening of the jig body 60. With this, the outer barrel 106 is sealed in an airtight manner when the jig body 60 is attached to the outer barrel 106 (specifically, when the jig body 60 is brought into contact with a proximal end surface 106a of a flange 117 of the outer barrel 106).

A gap 70 through which air is flowable is formed between an inner wall of the jig body 60 and the gasket 112 with the gasket 112 being stored in the storage space 64. Specifically, when the gasket 112 is stored in the storage space 64, and the production jig 52 is attached to the outer barrel 106, the storage space 64 and the inside of the outer barrel 106 (the space above the liquid level 110s of the drug solution 110 in the outer barrel 106) are communicated with each other via the gap 70.

The gap 70 is composed of a plurality of vent grooves 71 formed on the inner wall of the jig body 60. Due to the gap 70 (vent grooves 71) thus configured, air at the distal end of the gasket 112 and air at the proximal end of the gasket 112 can be communicated with each other. The number of the vent grooves 71 is not particularly limited. However, two to eight vent grooves 71 are preferably formed, and four to six vent grooves 71 are more preferably formed. In the embodiment illustrated in this figure, the vent grooves 71 extend along the moving direction (vertical direction) of the gasket 112. The vent 66 may extend in the direction diagonal to the moving direction of the gasket 112. In this case, the vent 66 may helically extend, for example.

The vent 66 is formed at the side of the jig body 60 and extends in the horizontal direction in Fig. 5 to penetrate through the wall (side wall) of the jig body 60.

The jig moving mechanism 54 moves the jig body 60 in the vertical direction under the control of the control unit 59. The jig moving mechanism 54 is not particularly limited. Examples of the jig moving mechanism include a fluid pressure cylinder device, a linear motor, and a direct operated mechanism using a rack and pinion and a rotation motor.

The pushing member 62 is disposed to be movable in the vertical direction along generally a center axis of the jig body 60. The pushing member 62 has generally a cylindrical shape (rod-like shape) in the present embodiment. Specifically, the pushing member 62 is inserted into an insertion hole 72, which is formed on the center axis of the jig body 60 to extend in the vertical direction, in an airtight manner so as to be slidable.

When the production device 50 is configured to produce a plurality of pre-filled syringes 100 from a plurality of sets of the syringe assembly 107 and the gasket 112, a number of production jigs 52 corresponding to the number of the sets are provided.

In the present embodiment, the pushing member 62 has a function of arranging the gasket 112 at the proximal end opening 104 of the outer barrel 106 and further pushing the gasket 112 toward the distal end in the outer barrel 106 by pushing the gasket 112, which is held by the jig body 60, toward the syringe assembly 107 held by the syringe holding member 16 by the activation of the actuator 56. Accordingly, the pushing member 62 has both a function as a gasket arranging mechanism 74 for sealing the proximal end opening 104 of the outer barrel 106 with the gasket 112 and a function as a pushing mechanism 76 for pushing the gasket 112 toward the barrel tip 102 into the outer barrel 106.

In the present embodiment, the pushing member 62 has a base portion 78 with relatively a large diameter and a pin portion 80 extending downward from the lower end of the base portion 78 and having relatively a small diameter. The pushing member 62 can be inserted into the outer barrel 106 from the proximal end opening 104 of the outer barrel 106 of the syringe assembly 107 held by the syringe holding member 16. A second seal member 82 that is in contact with the outer peripheral surface of the base portion 78 of the pushing member 62 over the entire circumference is formed on the inner periphery of the insertion hole 72. With this, the pushing member 62 is slidable in an airtight manner. The second seal member 82 may be arranged on the outer periphery of the base portion 78.

The thickness (outer diameter) of the pin portion 80 of the pushing member 62 is smaller than the inner diameter of the fitted concave portion 124 formed on the gasket 112. Therefore, the distal end of the pin portion 80 can be inserted into the fitted concave portion 124.

The actuator 56 moves the pushing member 62 vertically under the control of the control unit 59. The actuator 56 can move the pushing member 62 downward with a speed of 50 to 20000 mm/min. The actuator 56 is not particularly limited. Examples of the actuator include a fluidpressure cylinder device, a linear motor, and a direct operated mechanism using a rack and pinion and a rotation motor.

An actuator that can accurately control the operating positon of an output portion serving as a movable portion under the control of the control unit 59 is preferably used for the actuator 56. It is to be noted that another member or a power transmission mechanism that can transmit a motion of the actuator 56 to the pushing member 62 may be present between the actuator 56 and the pushing member 62.

The pressure control mechanism 58 includes an air line 84 with one end connected to the jig body 60, a vacuum source 86 connected to the other end of the air line 84, and a control valve 88 mounted on the air line 84. The air line 84 is communicated with the vent 66 formed on the jig body 60. The vacuum source 86 is not particularly limited. A vacuum pump or a combination of an ejector and a positive pressure source can be used, for example. A solenoid valve can be used for the control valve 88, for example.

Under the control of the control unit 59, the vacuum source 86 in the pressure control mechanism 58 is operated, and the control valve 88 is opened, whereby air in the storage space 64 is sucked via the air line 84. Thus, the pressure control mechanism 58 can reduce the pressure in the storage space 64 to a desired pressure. The pressure control mechanism 58 can also return the pressure in the storage space 64 to the atmospheric pressure by stopping the vacuum source 86. An atmosphere open port may be provided on the control valve 88. When the port of the control valve 88 communicating with the vacuum source 86 is shut off, the atmosphere open port may be opened to allow the storage space 64 to be open to the atmosphere to return the pressure in the storage space 64 to the atmospheric pressure. Alternatively, an atmosphere open line and the control valve 88 may separately be provided to the jig body 60.

The production device 50 according to the present embodiment is basically configured as described above, and its operation and effect will next be described. A process of inserting the gasket 112 to a predetermined positon (insertion position) in the outer barrel 106 of the syringe assembly 107 in the production process of the pre-filled syringe 100 will especially be described. The processes below carried out by the production device 50 are performed under the control of the control unit 59. However, the embodiment is not limited thereto, and some processes may manually be performed by an operator.

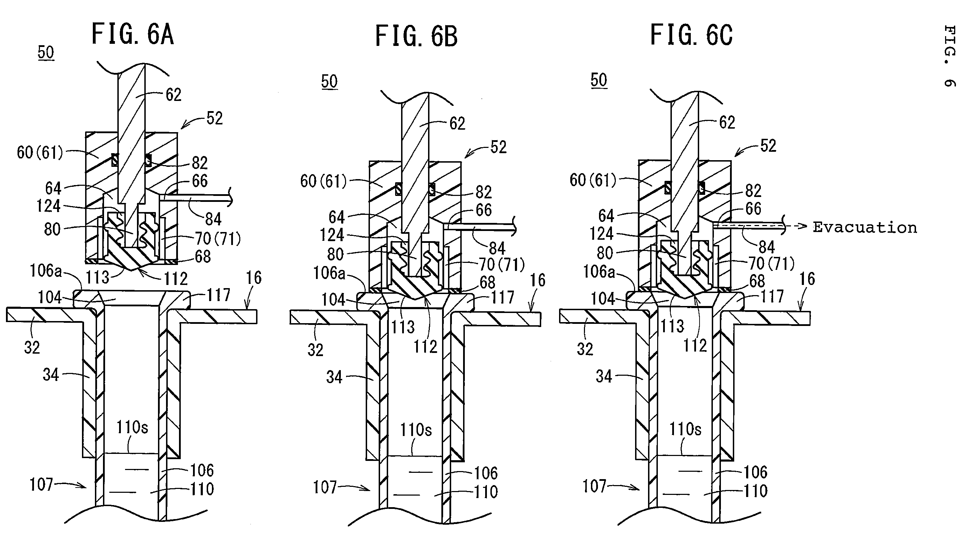

As illustrated in Fig. 6A, the syringe assembly 107 having the barrel tip 102 sealed with the cap 108 and having the outer barrel 106 filledwith a predetermined amount of the drug solution 110 is held with the barrel tip 102 being oriented at the bottom, and the gasket 112 is held at the opposite position (just above the proximal end opening 104) opposite to the proximal end opening 104 of the outer barrel 106. Specifically, the syringe assembly 107 is held by the syringe holding member 16, and the gasket 112 is held by the jig body 60.

Then, as illustrated in Fig. 6B, the jig body 60 is moved down by the jig moving mechanism 54, and attached to the outer barrel 106 so as to cover the proximal end opening 104 of the outer barrel 106. With this, the inside of the outer barrel 106 and the storage space 64 are isolated from the space outside the outer barrel 106 and the jig body 60, whereby the inside of the outer barrel 106 and the storage space 64 are formed as an airtight space. In addition, the storage space 64 and the inside of the outer barrel 106 (the space above the liquid level 110s of the drug solution 110) are communicated with each other via the gap 70.

Then, the pressure in the outer barrel 106 of the syringe assembly 107 held by the syringe holding member 16 is reduced to a set pressure. Specifically, the vacuum source 86 and the control valve 88 are operated to evacuate not only the storage space 64 but also the inside of the outer barrel 106 communicating with the storage space 64 via the gap 70 as illustrated in Fig. 6C. Thus, the pressure in the outer barrel 106 (the space above the liquid level 110s of the drug solution 110 in the outer barrel 106) is reduced to the set pressure.

The set pressure is a pressure set such that the pressure in the space between the liquid level 110s of the drug solution 110 and the distal end surface 113 of the gasket 112 becomes almost equal to the atmospheric pressure (760 mmHg) when the gasket 112 is inserted to the insertion position in the outer barrel 106. As described above, the set pressure can be calculated and set based on the above equation (1).

After the pressure in the outer barrel 106 is reduced to the set pressure, the proximal end opening 104 of the outer barrel 106 is sealed with the gasket 112. Specifically in the present embodiment, the gasket 112 held by the jig body 60 is pushed downward by the pushing member 62, while controlling the position of the pushing member 62 by driving the actuator 56 to move the pushing member 62 downward under the control of the control unit 59.

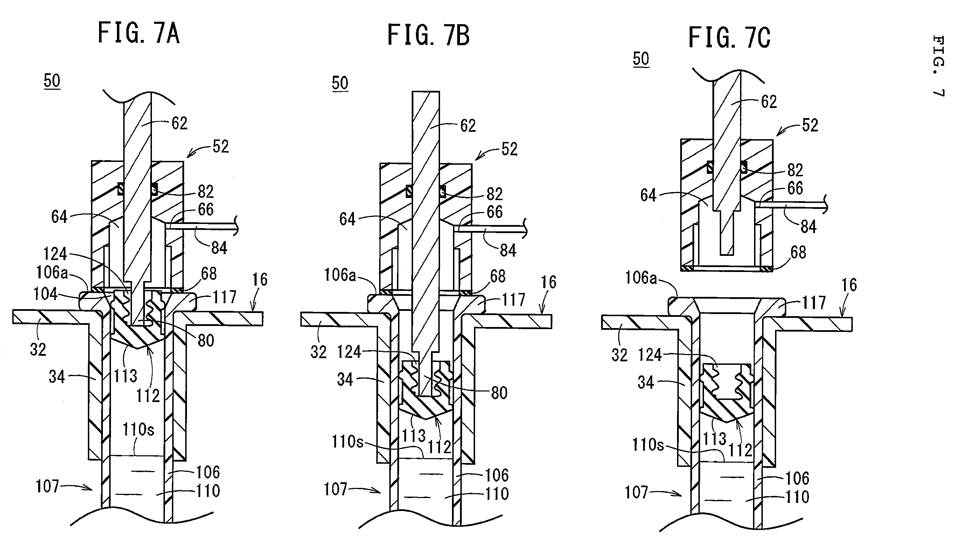

With this, the gasket 112 is detached from the jig body 60, and inserted into the proximal end opening 104 of the outer barrel 106, whereby the gasket 112 is disposed at the sealing position, as illustrated in Fig. 7A.

Then, the gasket 112 is pushed to the insertion position in the outer barrel 106 as illustrated in Fig. 7B. Specifically, the gasket 112 is pushed downward by the pushing member 62, while controlling the position of the pushing member 62 by driving the actuator 56 to move the pushing member 62 downward under the control of the control unit 59. In this case, in the present embodiment, the gasket 112 is pushed toward the barrel tip 102 with a liquid-tight sliding motion in the outer barrel 106 with a speed higher than a speed of moving the gasket 112 by utilizing the atmospheric pressure. For example, the gasket 112 is pushed into the outer barrel 106 with a speed of 50 to 20000 mm/min.

In this case, when the gasket 112 is moved to the insertion position (the position illustrated in Fig. 7B) in the outer barrel 106 from the position (the opposite position illustrated in Figs. 6B and 6C) where the gasket 112 is held by the jig body 60, the gasket 112 maybe pushed into the outer barrel 106 to the insertion position beyond the sealing position with the continuous motion of the pushing member 62 without being stopped at the sealing position (the position illustrated in Fig. 7A).

During the period in which the gasket 112 is pushed from the sealing position to the insertion position, the pressure in the storage space 64 and the pressure in the space above the gasket 112 in the outer barrel 106 may be kept reduced or may be returned to the atmospheric pressure.

The maximum speed of pushing the gasket 112 into the outer barrel 106 may be set to an upper limit of an operating speed of the actuator 56. The gasket 112 may be pushed with a speed not less than 20000 mm/min depending on the operating capability of the actuator 56. During the period in which the pushingmember 62 pushes to move the gasket 112 to the insertion position from the sealing position, the moving speed of moving the gasket 112, i.e., the moving speed of the pushing member 62, may be constant, or may be changed during the movement.

As illustrated in Fig. 7B, after the pushing member 62 pushes the gasket 112 to the insertion position in the outer barrel 106, the motion of the pushing member 62 is stopped. The pressure in the space between the drug solution 110 and the gasket 112 is increased due to the downward movement of the gasket 112 during the period in which the gasket 112 is moved from the sealing position to the insertion position. When the gasket 112 is inserted to the insertion position, the pressure in the space between the drug solution 110 in the outer barrel 106 and the gasket 112 becomes almost equal to the atmospheric pressure.

After the gasket 112 is inserted to the insertion position, the jig body 60 is moved upward, and the pushing member 62 is moved upward to be pulled out of the outer barrel 106 under the control of the control unit 59 as illustrated in Fig. 7C. When the storage space 64 and the space above the gasket 112 in the outer barrel 106 have a pressure-reduced state at the point at which the movement of the gasket 112 to the insertion position is completed, they are returned to be the atmospheric pressure, and then, the jig body 60 is moved upward.

Thus, the pre-filled syringe 100 illustrated in Fig. 1 is produced.

As described above, according to the production method for the pre-filled syringe 100 and the production device 50 of the present embodiment, the gasket 112 is forcibly pushed into the outer barrel 106 after being arranged at the sealing position relative to the outer barrel 106, when the sliding resistance of the gasket 112 to the outer barrel 106 is high. Therefore, as in the first embodiment, the gasket 112 can quickly be inserted to a predetermined position (insertion position) in the outer barrel 106. Accordingly, no problem is caused in a foreign matter inspection in a subsequent process, whereby production efficiency can be enhanced. In addition, the second embodiment can provide the operation and effect similar to the first embodiment.

The production device 50 may perform the operation of pushing the gasket 112 with a speed higher than the speed of moving the gasket 112 by using the atmospheric pressure during the whole process of moving the gasket 112 from the sealing position to the insertion position, or during a part of the process of moving the gasket 112 from the sealing position to the insertion position.

In the present embodiment, after being disposed at the sealing position by the pushing member 62, the gasket 112 is further pushed to the insertion position by the pushing member 62. However, the gasket 112 may be pushed to the insertion position with the process described below. Specifically, a pressure mechanism for increasing the pressure in the storage space 64 of the jig body 60 and the space above the gasket 112 in the outer barrel 106 to a desired pressure equal to or higher than the atmospheric pressure is provided to the production device 50. After the gasket 112 is arranged at the sealing position, the pressure in the storage space 64 of the jig body 60 and the space above the gasket 112 in the outer barrel 106 is increased to a pressure higher than the atmospheric pressure.

With this, the pressure higher than the atmospheric pressure is applied to the proximal end (upper side) of the gasket 112, so that the gasket 112 is pushed downward by the difference between the pressure higher than the atmospheric pressure and the pressure (reduced pressure) in the space between the drug solution 110 and the gasket 112. After the gasket 112 reaches the insertion position, the storage space 64 of the jig body 60 and the space above the gasket 112 in the outer barrel 106 are open to the atmosphere to return the pressure in the storage space 64 of the jig body 60 and the space above the gasket 112 in the outer barrel 106 to the atmospheric pressure, and the movement of the gasket 112 is stopped. The gasket 112 can also quickly be inserted to the insertion position by increasing the pressure in the storage space 64 and the space above the gasket 112 in the outer barrel 106 to a pressure higher than the atmospheric pressure as described above.

While the present invention has been described above with reference to the exemplary embodiments, the invention is not limited to the disclosed exemplary embodiments, and it is obvious that various modifications are possible without departing from the scope of the present invention.

## Claims

1. A production method for a pre-filled syringe that includes an outer barrel having a barrel tip and a proximal end opening, a cap sealing the barrel tip, a drug solution filled in the outer barrel, and a gasket that is slidable in the outer barrel, the gasket being in direct contact with an inner peripheral surface of the outer barrel, and sliding resistance of the gasket to the outer barrel being higher than sliding resistance of the gasket to the outer barrel if a liquid lubricant is present between the inner peripheral surface of the outer barrel and an outer peripheral surface of the gasket, the production method comprising:

   (a) holding a syringe assembly, which includes the barrel tip sealed with the cap and the outer barrel filled with a predetermined amount of the drug solution, with the barrel tip being oriented at a bottom;
   (b) reducing a pressure in the outer barrel of the syringe assembly held by the (a) to a set pressure;
   (c) sealing the proximal end opening of the outer barrel with the gasket while the pressure in the outer barrel is at the set pressure, thereby disposing the gasket to a sealing position; and
   (d) after the (c), pushing the gasket toward the barrel tip with a liquid-tight sliding motion in the outer barrel with a speed higher than a speed upon moving the gasket by utilizing the atmospheric pressure, in at least a part of a process of moving the gasket from the sealing position to an insertion position, the insertion position being closer to the barrel tip than the sealing position, and the insertion position being a position at which a distal end surface of the gasket is apart from a liquid level of the drug solution with a predetermined distance,

   wherein, at the insertion position, a pressure in a space between the liquid level of the drug solution and the distal end surface of the gasket is approximately equal to the atmospheric pressure.

2. The production method for a pre-filled syringe according to claim 1,
   wherein the maximum sliding resistance when the gasket slides from the sealing position to the insertion position in the outer barrel with a speed of 200 mm/min with the cap not attached to the barrel tip and with the drug solution not filled in the outer barrel is 3 N or higher, and
   wherein in the (d), the gasket is pushed into the outer barrel with a speed equal to or higher than 50 mm/min.

3. The production method for a pre-filled syringe according to claim 1,
   wherein in the (d), the gasket is pushed to the insertion position from the sealing position by controlling a position of a pushing member that is insertable into the outer barrel.

4. The production method for a pre-filled syringe according to claim 1, further comprising:

   (e) holding the gasket at an opposite position at which the distal end surface of the gasket opposes the proximal end opening of the outer barrel in the syringe assembly that is oriented with the barrel tip at the bottom,
   wherein, in the (d), the gasket located at the opposite position is pushed to the insertion position beyond the sealing position in the outer barrel by a continuous motion of the pushing member that is insertable into the outer barrel.

5. The production method for a pre-filled syringe according to claim 1,
   wherein, in the (d), only the gasket is in contact with a portion of the inner peripheral surface of the outer barrel at the side closer to the proximal end opening than to the drug solution.

6. The production method for a pre-filled syringe according to claim 1,
   wherein the set pressure is a pressure calculated based on a distance from a point where the gasket inserted from the proximal end opening first forms a liquid-tight seal with the inner peripheral surface of the outer barrel or from a proximal end surface of the outer barrel to a liquid level of the drug solution filled in the outer barrel that is oriented with the barrel tip at the bottom, and a distance from a most distal point where a liquid-tight seal is formed between the gasket at the insertion position and the inner peripheral surface of the outer barrel to the liquid level of the drug solution filled in the outer barrel that is oriented with the barrel tip at the bottom.

7. A pre-filled syringe production device for producing a pre-filled syringe that includes an outer barrel having a barrel tip and a proximal end opening, a cap sealing the barrel tip, a drug solution filled in the outer barrel, and a gasket that is slidable in the outer barrel, the gasket being in direct contact with an inner peripheral surface of the outer barrel, and sliding resistance of the gasket to the outer barrel being higher than sliding resistance of the gasket to the outer barrel if a liquid lubricant is present between the inner peripheral surface of the outer barrel and an outer peripheral surface of the gasket, the pre-filled syringe production device comprising:

   a syringe holding member configured to hold a syringe assembly, which includes the barrel tip sealed with the cap and the outer barrel filled with a predetermined amount of the drug solution, with the barrel tip oriented at the bottom;

a pressure control mechanism configured to reduce a pressure in the outer barrel of the syringe assembly to a set pressure;

a gasket arranging mechanism configured to dispose the gasket at a sealing position by sealing the proximal end opening of the outer barrel with the gasket; and

a pushing mechanism configured to push the gasket toward the barrel tip in the outer barrel, wherein the pushing mechanism is configured to push the gasket toward the barrel tip with a liquid-tight sliding motion in the outer barrel with a speed higher than a speed upon moving the gasket by utilizing the atmospheric pressure, in at least a part of a process of moving the gasket from the sealing position to an insertion position that is closer to the barrel tip than the sealing position and where a distal end surface of the gasket is apart from a liquid level of the drug solution with a predetermined distance, and wherein, at the insertion position, a pressure in a space between the liquid level of the drug solution and the distal end surface of the gasket is approximately equal to the atmospheric pressure.

8. The pre-filled syringe production device according to claim 7,
wherein the maximum sliding resistance when the gasket slides from the sealing position to the insertion position in the outer barrel with a speed of 200 mm/min with the cap not attached to the barrel tip and with the drug solution not filled in the outer barrel is 3 N or higher, and
wherein the pushing mechanism is configured to push the gasket into the outer barrel with a speed equal to or higher than 50 mm/min.

9. The pre-filled syringe production device according to claim 7, wherein the pushing mechanism is configured to push the gasket to the insertion position from the sealing position by controlling a position of a pushing member that is insertable into the outer barrel.

10. The pre-filled syringe production device according to claim 7, further comprising a gasket holding member that is configured hold the gasket at an opposite position at which the distal end surface of the gasket opposes the proximal end opening of the outer barrel in the syringe assembly that is oriented with the barrel tip at the bottom,
wherein a pushing member that is insertable into the outer barrel and that serves as both the gasket arranging mechanism and the pushing mechanism, the pushing member configured to push the gasket located at the opposite position to the insertion position beyond the sealing position in the outer barrel in a continuous motion of the pushing member.

11. The pre-filled syringe production device according to claim 7,
wherein only the gasket is in contact with a portion of the inner peripheral surface of the outer barrel at the side closer to the proximal end opening than to the drug solution when the gasket is moved to the insertion position from the sealing position.

12. The pre-filled syringe production device according to claim 7,
wherein the set pressure is a pressure calculated based on a distance from a point where the gasket inserted from the proximal end opening first forms a liquid-tight seal with the inner peripheral surface of the outer barrel or from a proximal end surface of the outer barrel to a liquid level of the drug solution filled in the outer barrel that is oriented with the barrel tip at the bottom, and a distance from a most distal position where a liquid-tight seal is formed between the gasket at the insertion position and the inner peripheral surface of the outer barrel to the liquid level of the drug solution filled in the outer barrel that is oriented with the barrel tip at the bottom.

FIG. 1

# FIG. 2

FIG. 3

FIG. 4A    FIG. 4B    FIG. 4C    FIG. 4D

FIG. 4

EP 2 995 333 A1

# FIG. 5

FIG. 6A  FIG. 6B  FIG. 6C

FIG. 6

EP 2 995 333 A1

FIG. 7

FIG. 7A    FIG. 7B    FIG. 7C

**INTERNATIONAL SEARCH REPORT**

International application No.

PCT/JP2013/063210

| A. CLASSIFICATION OF SUBJECT MATTER |
|---|
| *A61M5/28*(2006.01)i |

According to International Patent Classification (IPC) or to both national classification and IPC

| B. FIELDS SEARCHED |
|---|
| Minimum documentation searched (classification system followed by classification symbols) |
| A61M5/28 |

Documentation searched other than minimum documentation to the extent that such documents are included in the fields searched

| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)

| C. DOCUMENTS CONSIDERED TO BE RELEVANT | | | |
|---|---|---|---|
| Category* | Citation of document, with indication, where appropriate, of the relevant passages | | Relevant to claim No. |
| Y | JP 2008-212213 A (Hisamitsu Pharmaceutical Co., Inc.), 18 September 2008 (18.09.2008), paragraphs [0014] to [0029]; fig. 1 to 2 (Family: none) | | 1-12 |
| Y | JP 09-299480 A (Eisai Co., Ltd.), 25 November 1997 (25.11.1997), paragraphs [0005] to [0006], [0028] to [0046]; fig. 7 to 12 (Family: none) | | 1-12 |

☐ Further documents are listed in the continuation of Box C.  ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 26 July, 2013 (26.07.13) | 06 August, 2013 (06.08.13) |

| Name and mailing address of the ISA/ | Authorized officer |
|---|---|
| Japanese Patent Office | |
| Facsimile No. | Telephone No. |

Form PCT/ISA/210 (second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4028386 B